# FASCICULE DE BREVET EUROPEEN

(11) **EP 2 872 072 B1**
(45) Date de publication et mention de la délivrance du brevet: **18.01.2017**
(21) Numéro de dépôt: 13745454.2
(22) Date de dépôt: 11.07.2013
(51) Int. Cl.: A61F 2/34

(54) **COTYLE POUR PROTHESE DE HANCHE**
GELENKPFANNE FÜR EINE HÜFTPROTHESE
ACETABULUM FOR A HIP PROSTHESIS

(30) Priorité: 11.07.2012 FR 1256662
(43) Date de publication de la demande: 20.05.2015
(73) Titulaire: RV Finances, 69140 Rillieux-la-Pape (FR)
(72) Inventeur: VANDEVELDE, Richard, 69140 Rillieux-la-Pape (FR); BIGNON, Aurélien, 69300 Caluire Et Cuire (FR); CORDIER, Delphine, 13100 Aix en Provence (FR)
(74) Mandataire: Weber, Jean-François
(86) Numéro de dépôt international: PCT/FR2013/051668
(87) Numéro de publication internationale: WO 2014/009669

(56) Documents cités:
- EP-A1- 1 216 667
- EP-A1- 1 647 242
- DE-A1-102008 044 951
- FR-A1- 2 929 104
- FR-A1- 2 950 524

## Description

### DOMAINE TECHNIQUE

La présente invention concerne un cotyle pour prothèse de hanche.

### TECHNIQUE ANTERIEURE

La majorité des cotyles sont constitués d'une cupule métallique assurant l'interface avec l'os et d'un insert assurant le contact avec la tête fémorale, la cupule et l'insert pouvant être ou non cimentés entre eux. Il s'agit généralement du ciment PMMA (Polyméthacrylate de méthyle). Ces prothèses présentent un excellent taux de survie à 10 ans (environ 95% d'entre elles). Cependant, passée cette décennie, le risque de descellement de la prothèse s'accroît de manière exponentielle.

Peu de cotyles présentent un taux de survie supérieur à 20 ans, et ce pour plusieurs raisons, dont notamment :
- La rigidité de la cupule peut favoriser une usure à son interface avec l'insert.
- Les débris issus de l'usure et des frottements entre les pièces du cotyle ne sont pas sans conséquences graves pour la santé du patient, puisqu'ils peuvent engendrer une ostéolyse et un descellement de l'implant.
- La cupule métallique étant trop rigide par rapport à l'os, une micro-mobilité peut être générée à l'interface, ainsi qu'une atrophie osseuse par dérivation des contraintes.
- Plus la cupule métallique est épaisse, moins il reste d'espace disponible pour l'insert, et par conséquent, plus l'épaisseur de ce dernier doit être réduite. Cela a pour effet d'augmenter les contraintes dans l'insert, et donc d'en accélérer l'usure.

Pour pallier ces inconvénients, des cotyles dits « *monoblocs non cimentés »* (à savoir que le revêtement de la cupule est directement fixé sur l'insert) ont été mis au point. Ils sont réalisés en un polymère biocompatible tel que du polyéthylène (par exemple l'UHMWPE « *Ultra-high-molecular-weight polyethytene »),* sur lequel a été directement déposé un revêtement en titane pur ou un alliage de titane. Grâce à cette innovation, les cotyles sont plus durables, car ils résolvent les problèmes dus à la rigidité des cupules métalliques détaillés ci-dessus. En effet, l'ensemble du cotyle conserve la rigidité du polyéthylène, qui est proche de celle de l'os.

De plus, le fait de réaliser un cotyle monobloc (insert en polyéthylène revêtu de titane) permet d'augmenter l'épaisseur du polyéthylène, par comparaison avec un insert qui est couplé avec une cupule métallique. Cela procure l'avantage de soulager les contraintes mécaniques qui sont infligées à la cupule et de rallonger la durée de vie du cotyle.

Cependant, ces cotyles « *monoblocs non cimentés* », présents à ce jour sur le marché, ne sont pas entièrement satisfaisants du point de vue du risque de relargage de particules de titane pendant et après la pose du cotyle dans le corps du patient. Plus précisément, le risque du relargage des particules de titane peut se produire lors de la sollicitation mécanique au cours de la pose du cotyle ou bien lors de frottements dus aux mouvements du patient. Ces particules peuvent alors migrer dans le corps, notamment dans les parties frottantes de l'implant et engendrer une usure catastrophique de l'implant.

Le document FR-2 950 524 divulgue un implant cotyloidien comportant une bague métallique ainsi qu'une cupule en polymère biocompatible, la partie polaire de la cupule et la zone de jonction entre la partie polaire et la bague étant recouverte d'un premier revêtement poreux comprenant du titane et d'un second revêtement de phosphate de calcium, tel que de l'hydroxyapatite, disposé sur le premier revêtement.

### EXPOSE DE L'INVENTION

La présente invention surmonte ces inconvénients inhérents aux cotyles monoblocs non cimentés connus à ce jour en proposant de nouveaux cotyles monoblocs non cimentés, réalisés en un polymère biocompatible et dont le revêtement a été optimisé pour présenter un risque amoindri de relargage de particules de titane pendant et après leur pose dans le corps du patient par rapport aux cotyles monoblocs non cimentés actuellement sur le marché. En outre, selon certains modes de réalisation de l'invention, les cotyles selon l'invention présentent aussi l'avantage non négligeable d'être plus ostéoconducteurs que les cotyles monoblocs non cimentés connus à ce jour et présents sur le marché.

La présente invention a pour premier objet un cotyle pour prothèse de hanche en un polymère biocompatible, ledit cotyle étant recouvert d'un revêtement qui comprend une première couche de titane qui a été appliquée par compression à chaud sur ledit polymère biocompatible, ledit revêtement se caractérise en ce que :
- la rugosité Rt de la première couche de titane est comprise entre 40 et 500 µm, de manière préférée entre 100 et 500 µm, ou encore entre 100 et 200 µm,
- ledit revêtement comprend en outre sur la première couche :
   - une deuxième couche de titane ou d'un phosphate de calcium ;
   - optionnellement, sur ladite deuxième couche, lorsque la deuxième couche est une couche de titane, une troisième couche d'un phosphate de calcium.

L'invention a aussi pour objet un procédé de fabrication dudit cotyle.

### MEILLEURE MANIERE DE REALISER L'INVENTION

Dans le cadre de la présente invention, on entend par « *couche de tatane » :*
- soit une couche constituée essentiellement de titane pur,
- soit une couche constituée essentiellement d'un alliage de titane. De manière préférée, il s'agit de l'alliage TA6V (alliage dont la composition en poids est : titane 90% ; aluminium 6% et vanadium : 4%) ;

De manière avantageuse, le titane utilisé pour réaliser la première couche du revêtement du cotyle se présente sous la forme d'une poudre dont la granulométrie est comprise entre 20 à 500 µm.

Dans le cadre de l'invention, on entend par « *couche d'un phosphafe de calcium »,* une couche constituée essentiellement d'au moins un phosphate de calcium, éventuellement couplé avec un verre bioactif.

De manière préférée, le phosphate de calcium est de l'hydroxyapatite, de la brushite ou bien un mélange d'hydroxyapatite et de brushite.

Dans le cadre de la présente invention, on entend par polymère biocompatible, un polymère qui puisse être implanté dans le corps humain sans qu'il ne soit rejeté par le patient ou provoque des réactions néfastes.

De plus, les polymères biocompatibles utilisés dans le cadre de l'invention présentent :
- une résistance mécanique telle que le cotyle assure sa fonction de prothèse une fois qu'il a été implanté dans le corps du patient au niveau de la hanche,
- une résistance thermique telle que ledit polymère biocompatible résiste aux températures auxquelles il est soumis pendant l'application des différentes couches du revêtement.

Avantageusement, le polymère biocompatible est choisi dans le groupe constitué par le polyéthylène à très haute masse moléculaire (abrégé *« UHMWPE* »), les polyaryléthercétones (abrégé « *PAEK* ») tels que les polyétheréthercétones (PEEK).

De manière préférée, le polymère biocompatible est le polyéthylène à très haute masse moléculaire (abrégé « *UHMWPE »).*

De plus, l'UHMWPE peut être non réticulé, réticulé, éventuellement chargé avec des antioxydants. Les antioxydants sont avantageusement choisis parmi les caroténoïdes, les vitamines A, B, C, E (tocophérols) et K, les polyphénols, le BHT, les gallates de propyle, d'octyle et de dodécyle, l'acide lipoïque, l'acide dihydroxylipoïque, les amines, l'hydroquinone, la coenzyme Q10 et le glutathion.

L'UHMWPE est éventuellement chargé en carbone. Le carbone peut avoir été mélangé dans l'UHMWPE. Ou bien, l'UHMWPE se trouve sous la forme d'un bi-couches, à savoir qu'une première couche a été chargée en carbone et la deuxième couche est constituée d'UHMWPE sous forme pure.

Selon un mode de réalisation de l'invention, le polymère biocompatible est du PEEK chargé en carbone.

La mesure de la rugosité permet d'apprécier un état d'une surface. Elle quantifie les variations de hauteur d'un profil de la surface. Dans le cadre de la présente invention, la rugosité est un paramètre physique important, car l'accroche du cotyle à l'os dépend de la rugosité du cotyle. La rugosité participe à l'accroche sur l'os. La mise en oeuvre d'une rugosité Rt suffisante, comprise entre 40 et 500 µm, pour la première couche de titane, contribue en outre à isoler thermiquement le polymère biocompatible de la chaleur susceptible d'être dégagée par le dépôt de la deuxième couche, par exemple lorsque cette dernière est appliquée par projection thermique (par exemple plasma chaud). Dans le cadre de la présente invention, on entend par rugosité totale Rt, la somme de la plus grande des hauteurs de saillie du profil et de la plus grande des profondeurs de creux du profil, à l'intérieur de la longueur d'évaluation, comme le définit la norme ISO4287.

Plus précisément, pour mesurer la rugosité de la dernière couche de revêtement du cotyle, lorsqu'il s'agit d'une couche de titane pur ou d'alliage de titane, on effectue les étapes suivantes :
- On découpe les cotyles selon leur tranche, puis on les enrobe dans une résine telle qu'une résine à base de méthyleméthacrylate (par exemple : la RESINE 605 commercialisée par la société LAM PLAN S.A.) de manière à protéger le revêtement pendant l'étape suivante de polissage.
- On effectue un polissage de la tranche pour avoir une surface nette observable.
- On réalise une série de photographies avec un microscope.
- On analyse les photographies à l'aide d'un logiciel de traitement d'images pour mesurer la rugosité de la dernière couche de revêtement du cotyle.

Pour mesurer la rugosité d'une couche de revêtement du cotyle lorsqu'il s'agit d'une couche de phosphate de calcium, on utilise un rugosimètre. Pour ce faire, on déplace un palpeur (pièce destinée à toucher la surface) au contact de la surface mesurée. Le rugosimètre enregistre les variations de hauteur le long du déplacement du palpeur de manière à obtenir une valeur de la rugosité.

L'application de la première couche de titane du revêtement du cotyle par compression à chaud peut être effectuée selon un cycle, selon les paramètres physiques suivants :
- une mise sous pression entre 20 et 100 bars dans une chambre de compression.
- Une élévation de la température jusqu'à environ 200°C.

La technique de la compression à chaud pour réaliser une première couche de revêtement de titane sur un cotyle en un polymère biocompatible est parfaitement à la portée de l'homme du métier.

L'épaisseur de la première couche du revêtement du cotyle est avantageusement comprise entre 50 et 800 µm, de manière préférée entre 150 et 500 µm, et de façon encore plus préférentielle entre 150 et 250 µm.

De manière avantageuse, lorsque le titane utilisé pour réaliser la première couche du revêtement du cotyle a une granulométrie dite « *standard »* (à savoir comprise entre environ 90 et 125 µm), la rugosité de la première couche de titane obtenue à l'issue de la compression à chaud est comprise entre 80 µm à 160 µm. Le taux de recouvrement de la première couche de titane sur le polymère biocompatible est alors avantageusement compris entre 60 et 80%. L'épaisseur de cette première couche du revêtement du cotyle est alors comprise entre 150 µm et 500 µm, et de façon encore plus préférentielle entre 150 et 250 µm.

Lorsque le titane utilisé pour réaliser la première couche du revêtement du cotyle a une granulométrie plus importante, à savoir comprise entre 200 et 500 µm environ, la rugosité de la première couche de titane obtenue à l'issue de la compression à chaud est comprise entre 180 et 500 µm. Le taux de recouvrement de la première couche de titane sur le polymère biocompatible est alors avantageusement compris entre 70 et 90%. L'épaisseur de cette première couche du revêtement du cotyle est alors comprise entre 300 et 800 µm.

Le taux de recouvrement de la première couche du revêtement du cotyle sur le polymère biocompatible dépend à la fois de l'épaisseur de la première couche de titane appliquée et de sa granulométrie. Le taux de recouvrement est avantageusement d'au moins 60%. Selon un mode de réalisation de l'invention, le taux de recouvrement est compris entre 60 et 80%.

De manière particulièrement avantageuse, le taux de recouvrement est quasiment de 100%.

La première couche de titane est relativement facile à arracher du polymère biocompatible du cotyle, car chaque grain de titane est ancré individuellement dans le polymère biocompatible. Le grain de titane comporte des aspérités. Pendant la phase de compression à chaud, le polymère biocompatible fond autour du grain de titane en se logeant dans ces aspérités. Le grain de titane est donc retenu par la force mécanique qu'exerce le polymère biocompatible à son contact. Ainsi, pour qu'un grain de titane se descelle, il faudrait que cette force soit suffisante pour déformer le polymère biocompatible de manière à le libérer du polymère biocompatible.

Les particules de titane ancrées dans le polymère biocompatible forment un revêtement rugueux.

La première couche du revêtement du cotyle a les fonctions suivantes :
- Elle assure l'accroche du revêtement sur le polymère biocompatible.
- Elle sert de support pour la deuxième couche du revêtement du cotyle selon l'invention.

Ainsi, la rugosité de la première couche du revêtement du cotyle est une caractéristique essentielle de la présente invention, car cette première couche sert de support à la deuxième couche du revêtement du cotyle selon l'invention. Si la première couche n'est pas suffisamment rugueuse, le dépôt de la deuxième couche du revêtement du cotyle selon l'invention ne peut pas être réalisé correctement.

La rugosité de la deuxième couche du revêtement du cotyle est avantageusement comprise entre 30 et 400 µm, de préférence entre 100 et 400 µm, ou de façon préférentielle entre 100 et 180 µm.

L'épaisseur de la deuxième couche du revêtement du cotyle peut être comprise entre 5 et 500 µm, de préférence entre 10 et 400 µm, ou de façon préférentielle entre 10 et 250 µm.

Plus précisément, l'épaisseur de la deuxième couche du revêtement du cotyle selon l'invention est avantageusement comprise entre 5 et 200 µm, lorsque la deuxième couche est une couche de phosphate de calcium.

L'épaisseur de la deuxième couche du revêtement du cotyle selon l'invention est avantageusement comprise entre 50 et 500 µm, lorsque la deuxième couche est une couche de titane.

Selon un mode de réalisation avantageux de l'invention, lorsqu'il s'agit d'une deuxième couche de titane, la deuxième couche est appliquée par projection plasma, et par exemple par projection plasma chaud.

Lorsqu'il s'agit d'une deuxième couche de phosphate de calcium, la deuxième couche du revêtement du cotyle est avantageusement appliquée au moyen de l'une des techniques suivantes:
- projection plasma,
- électrodéposition,
- trempage dans un bain de phosphate de calcium.

Il est à noter que lorsqu'on réalise la deuxième couche du revêtement du cotyle par projection plasma, l'épaisseur de la première couche doit être suffisamment importante pour isoler le polymère biocompatible (qui est sensible à la température) du matériau (titane, alliage de titane tel que le TA6V ou phosphate de calcium) projeté par projection plasma. En effet, ledit matériau est en fusion au moment du dépôt par projection plasma. C'est pourquoi, il est nécessaire qu'il ne soit pas en contact direct avec le polymère biocompatible pour ne pas le dégrader. Ainsi, il convient que l'épaisseur de la première couche du revêtement du cotyle soit suffisamment épaisse pour assurer une bonne isolation du polymère biocompatible lors d'une projection plasma. La mise en oeuvre d'une rugosité Rt comprise entre 40 et 500 µm, et de façon encore plus préférentielle entre 100 et 500 µm, ou entre 100 et 200 µm contribue à cette isolation thermique, en permettant de maintenir le matériau en fusion destiné à former la deuxième couche à une distance suffisante du polymère biocompatible, ce qui évite de dégrader ce dernier.

Le trempage consiste à plonger le cotyle revêtu de la première couche de titane dans un bain de phosphate de calcium, à savoir dans une solution qui contient un taux de phosphate de calcium de l'ordre de 40% en volume. Le phosphate de calcium contenu dans la solution se fixe alors sur la première couche de titane de manière à constituer une deuxième couche de revêtement du cotyle.

En ce qui concerne la technique par électrodéposition, le revêtement de phosphate de calcium obtenu par cette technique présente les avantages d'être d'épaisseur constante et présent même dans les porosités de la première couche de titane.

La première couche du revêtement du cotyle est avantageusement constituée d'une multitude de grains de titane qui sont individuellement scellés dans le polymère biocompatible. C'est pourquoi, la contrainte ponctuelle nécessaire au descellement de ces grains de titane est relativement peu élevée.

La seconde couche du revêtement du cotyle se superpose à la première couche.

En revanche, la seconde couche est répartie de manière continue (car les particules de titane pur, d'alliage de titane tel que le TA6V ou de phosphate de calcium sont soudées entre elles) à la surface de la première couche. Quelle que soit l'une des techniques d'application précitées, la deuxième couche se forme au contact des grains de titane pur ou d'alliage de titane tel que le TA6V de la première couche du revêtement du cotyle par logement, du matériau de ladite deuxième couche dans les aspérités desdits grains de la première couche.

Du fait que la seconde couche soit sensiblement continue, la même force ponctuelle appliquée à la surface du revêtement engendre des contraintes différentes, puisqu'elles vont se répartir dans toute la deuxième couche. Plus précisément, la deuxième couche étant en contact avec sensiblement tous les grains de titane de la première couche, la surface de contact qui subit la contrainte est beaucoup plus grande. C'est pourquoi, il faudrait une force beaucoup plus importante pour provoquer le descellement de la deuxième couche de revêtement du cotyle que pour provoquer le descellement des grains de titane de la première couche, car il s'agit dans le cas de la deuxième couche de rompre des liaisons métalliques (si le matériau de la deuxième couche est du titane pur et/ou un alliage de titane tel que TA6V) ou des liaisons covalentes (si le matériau de la deuxième couche est du phosphate de calcium).

Le contact privilégié du matériau de la deuxième couche avec les grains de titane de la première couche de revêtement du cotyle empêche cette deuxième couche de se décrocher du cotyle.

Ainsi, la deuxième couche de revêtement du cotyle selon l'invention (en titane pur, et/ou en alliage de titane tel que le TA6V ou encore en phosphate de calcium) renforce la première couche en engendrant un revêtement du cotyle plus robuste que si le cotyle n'était revêtu que d'une première couche de titane.

De plus, lorsque la deuxième couche du revêtement du cotyle est constituée de phosphate de calcium, cela présente en outre l'avantage que le cotyle selon l'invention présente un revêtement qui assure un support de croissance stable pour l'os.

On comprend alors tout l'intérêt de cette deuxième couche du revêtement du cotyle selon l'invention.

Optionnellement, le revêtement du cotyle selon l'invention comprend une troisième couche de phosphate de calcium lorsque la deuxième couche est une couche de titane

L'épaisseur de cette troisième couche est avantageusement comprise entre 5 et 200 µm.

La troisième couche est appliquée sur ladite deuxième couche de manière préférée par:
- projection plasma,
- électrodéposition,
- trempage dans un bain de phosphate de calcium.

La troisième couche permet d'obtenir un cotyle ostéoconducteur.

Dans un mode de réalisation particulièrement avantageux, l'invention concerne ainsi un cotyle revêtu d'une première couche de titane, elle-même recouverte d'une deuxième couche de titane, elle-même recouverte d'une troisième couche de phosphate de calcium.

L'invention a aussi pour objet un procédé de fabrication d'un cotyle tel que décrit ci-dessus. Le procédé de fabrication comprend les étapes suivantes :
a) on dispose d'un cotyle en un matériau biocompatible, par exemple en UHMPWE ou en PEAK ;
b) on revêt ledit cotyle d'une première couche de titane pur ou d'un alliage de titane tel que le TA6V par compression à chaud ;
c) on revêt ladite première couche de titane d'une deuxième couche, ladite deuxième couche étant une couche de titane ou une couche d'un phosphate de calcium ;
d) optionnellement, lorsque la deuxième couche est une couche de titane, on revêt ladite deuxième couche d'une troisième couche, ladite troisième couche étant une couche d'un phosphate de calcium.

Selon un mode de réalisation du procédé de fabrication, la deuxième couche est une couche d'un phosphate de calcium et a été appliquée sur la première couche par projection plasma, électrodéposition, ou trempage dans un bain de phosphate de calcium.

Selon un autre mode de réalisation du procédé de fabrication, la deuxième couche est une couche de titane et a été appliquée par projection plasma sur la première couche.

Ainsi, l'invention met en oeuvre, dans une variante préférentielle de réalisation, un dépôt par compression à chaud de grains de titane à la surface d'un cotyle en polymère biocompatible, de façon à former ainsi à la surface dudit cotyle une première couche de titane présentant une rugosité comprise entre 40 et 500 µm, puis une projection thermique (plasma par exemple) d'un matériau pulvérulent (par exemple une poudre de titane), de telle sorte que ladite poudre ainsi projetée vienne former une deuxième couche sur la première couche, tout en fusionnant avec les grains de titane formant la première couche, et ce sans altérer le polymère biocompatible qui est protégé thermiquement, en particulier par la rugosité de la première couche, de la chaleur issue de la poudre en fusion projetée sur la première couche.

L'invention permet ainsi de réduire le risque de voir les grains de titane se détacher du polymère biocompatible, puisque lesdits grains sont avantageusement liés entre eux par une deuxième couche (de titane fondu par exemple), de préférence sensiblement continue, et ce sans qu'il n'ait été nécessaire d'augmenter la quantité surfacique de grains et sans que le polymère biocompatible n'ait été altéré thermiquement.

### POSSIBILITE D'APPLICATION INDUSTRIELLE

L'invention trouve son application industrielle dans la conception, la fabrication et l'utilisation de cotyles.

## Revendications

1. Cotyle pour prothèse de hanche en un polymère biocompatible, ledit cotyle étant recouvert d'un revêtement qui comprend une première couche de titane appliquée sur ledit polymère biocompatible et qui comprend en outre, sur la première couche, une deuxième couche de titane ou d'un phosphate de calcium, **caractérisé en ce que** :
- la rugosité Rt de la première couche de titane est comprise entre 40 et 500 µm,
- ladite première couche de titane a été appliquée par compression à chaud sur ledit polymère biocompatible.

2. Cotyle selon la revendication 1 **caractérisé en ce que** la rugosité Rt de la première couche de titane est comprise entre 100 et 500 µm.

3. Cotyle selon la revendication 1 ou 2 **caractérisé en ce que** ledit revêtement comprend sur ladite deuxième couche, lorsque la deuxième couche est une couche de titane, une troisième couche d'un phosphate de calcium.

4. Cotyle selon l'une des revendications 1 à 3, **caractérisé en ce que** le polymère biocompatible est choisi dans le groupe constitué par le polyéthylène à très haute masse moléculaire (UHMWPE) non réticulé, réticulé et/ou chargé avec des antioxydants choisis de préférence parmi les caroténoïdes, les vitamines A, B, C, E (tocophérols) et K, les polyphénols, le BHT, les gallates de propyle, d'octyle et de dodécyle, l'acide lipoïque, l'acide dihydroxylipoïque, les amines, l'hydroquinone, la coenzyme Q10 et le glutathion, les polyaryléthercétones (PAEK), tels que les polyétheréthercétones (PEEK) chargés ou non en carbone.

5. Cotyle selon l'une quelconque des revendications précédentes, **caractérisé en ce que** l'épaisseur de la première couche de titane est comprise entre 50 et 800 µm.

6. Cotyle selon la revendication 5 **caractérisé en ce que** l'épaisseur de la première couche de titane est comprise entre 150 et 500 µm.

7. Cotyle selon l'une quelconque des revendications précédentes, **caractérisé en ce que** le taux de recouvrement de la première couche de titane sur le polymère biocompatible est d'au moins 60%.

8. Cotyle selon l'une quelconque des revendications précédentes, **caractérisé en ce que** la rugosité de la deuxième couche est comprise entre 30 et 400 µm.

9. Cotyle selon la revendication 8 **caractérisé en ce que** la rugosité de la deuxième couche est comprise entre 100 et 400 µm.

10. Cotyle selon l'une quelconque des revendications précédentes, **caractérisé en ce que** l'épaisseur de la deuxième couche du revêtement du cotyle est comprise entre 5 et 500 µm.

11. Cotyle selon la revendication 10 **caractérisé en ce que** l'épaisseur de la deuxième couche du revêtement du cotyle est comprise entre 10 et 400 µm.

12. Cotyle selon l'une quelconque des revendications 1 à 11, **caractérisé en ce que** le revêtement dudit cotyle comprend une deuxième couche de titane et une troisième couche en un phosphate de calcium dont l'épaisseur est comprise entre 5 et 200 µm.

13. Cotyle selon l'une des revendications précédentes **caractérisé en ce que** ladite première couche est constituée d'une multitude de grains de titane qui sont individuellement scellés dans le polymère biocompatible.

14. Cotyle selon la revendication 13 **caractérisé en ce que** ladite deuxième couche est sensiblement continue et est en contact avec sensiblement tous les grains de titane de la première couche.

15. Procédé de fabrication d'un cotyle selon l'une quelconque des revendications 1 à 14, ledit procédé de fabrication comprenant les étapes suivantes :
a) on dispose d'un cotyle en un matériau biocompatible, par exemple en UHMPWE ou en PEAK ;
b) on revêt ledit cotyle d'une première couche de titane ;
c) on revêt ladite première couche de titane d'une deuxième couche, ladite deuxième couche étant une couche de titane ou une couche d'un phosphate de calcium ;
ledit procédé étant **caractérisé en ce qu'**on revêt ledit cotyle de ladite première couche de titane par compression à chaud, la rugosité Rt de la première couche de titane étant comprise entre 40 et 500 µm.

16. Procédé de fabrication d'un cotyle selon la revendication 15 **caractérisé en ce qu'**il comprend un étape d) dans laquelle, lorsque la deuxième couche est une couche de titane, on revêt ladite deuxième couche d'une troisième couche, ladite troisième couche étant une couche d'un phosphate de calcium.

17. Procédé de fabrication d'un cotyle selon la revendication 16 **caractérisé en ce que** la troisième couche a été appliquée sur la deuxième couche par projection plasma, électrodéposition, ou trempage dans un bain de phosphate de calcium.

18. Procédé de fabrication d'un cotyle selon l'une des revendications 15 à 17, **caractérisé en ce que** la deuxième couche est une couche d'un phosphate de calcium et a été appliquée sur la première couche par projection plasma, électrodéposition, ou trempage dans un bain de phosphate de calcium.

19. Procédé de fabrication d'un cotyle selon l'une des revendications 15 à 17, **caractérisé en ce que** la deuxième couche est une couche de titane et a été appliquée par projection plasma sur la première couche.

20. Procédé de fabrication d'un cotyle selon l'une des revendications 15 à 19 **caractérisé en ce que** l'étape b) au cours de laquelle on revêt ledit cotyle de ladite première couche de titane est réalisée par compression à chaud d'une multitude de grains de titane sur ledit polymère biocompatible, de façon que lesdits grains soient individuellement scellés dans le polymère biocompatible.

## Patentansprüche

1. Pfanne für Hüftprothese aus einem biologisch verträglichen Polymer, wobei die Pfanne mit einer Beschichtung versehen ist, die eine erste Titanschicht umfasst, die auf das biologisch verträgliche Polymer aufgetragen ist, und die außerdem auf der ersten Schicht eine zweite Titan- oder Kalziumphosphatschicht umfasst, **dadurch gekennzeichnet, dass**:
- die Rauheit Rt der ersten Titanschicht zwischen 40 und 500 µm liegt,
- die erste Titanschicht durch Heißpressen auf das biologisch verträgliche Polymer aufgebracht wurde.

2. Pfanne nach Anspruch 1, **dadurch gekennzeichnet, dass** die Rauheit Rt der ersten Titanschicht zwischen 100 und 500 µm liegt.

3. Pfanne nach Anspruch 1 oder 2, **dadurch gekennzeichnet, dass** die Beschichtung auf der zweiten Schicht - sofern die zweite Schicht eine Titanschicht ist - eine dritte Schicht aus Kalziumphosphat umfasst.

4. Pfanne nach einem der Ansprüche 1 bis 3, **dadurch gekennzeichnet, dass** das biologisch verträgliche Polymer aus der Gruppe von ultrahochmolekulargewichtigem Polyethylen (UHMWPE) stammt, das nicht vernetzt, vernetzt und/oder mit Antixydantien verstärkt ist, die bevorzugt unter den Carotenoiden, den Vitaminen A, B, C, E (Tocopherole) und K, den Polyphenolen, BHT, den Propyl-, Octyl- und Dodecylgallaten, der Liponsäure, der Dihydroxy-Liponsäure, den Aminen, dem Hydroquinon, dem Coenzym Q10 und dem Glutathion, den Polyaryletherketonen (PAEK), wie den mit Kohlenstoff verstärkten oder nicht mit Kohlenstoff verstärkten Polyetheretherketonen (PEEK), ausgewählt werden.

5. Pfanne nach irgendeinem der vorangehenden Ansprüche, **dadurch gekennzeichnet, dass** die Stärke der ersten Titanschicht zwischen 50 und 800 µm liegt.

6. Pfanne nach Anspruch 5, **dadurch gekennzeichnet, dass** die Stärke der ersten Titanschicht zwischen 150 und 500 µm liegt.

7. Pfanne nach irgendeinem der vorangehenden Ansprüche, **dadurch gekennzeichnet, dass** die Überdeckungsrate der ersten Titanschicht auf dem biologisch verträglichen Polymer zumindest 60% beträgt.

8. Pfanne nach irgendeinem der vorangehenden Ansprüche, **dadurch gekennzeichnet, dass** die Rauheit der zweiten Titanschicht zwischen 30 und 400 µm liegt.

9. Pfanne nach Anspruch 8, **dadurch gekennzeichnet, dass** die Rauheit der zweiten Titanschicht zwischen 100 und 400 µm liegt.

10. Pfanne nach irgendeinem der vorangehenden Ansprüche, **dadurch gekennzeichnet, dass** die Stärke der zweiten Beschichtungsschicht der Pfanne zwischen 5 und 500 µm liegt.

11. Pfanne nach Anspruch 10, **dadurch gekennzeichnet, dass** die Stärke der zweiten Beschichtungsschicht der Pfanne zwischen 10 und 400 µm liegt.

12. Pfanne gemäß irgendeinem der Ansprüche 1 bis 11, **dadurch gekennzeichnet, dass** die Beschichtung der Pfanne eine zweite Titanschicht und eine dritte Schicht aus einem Kalziumphosphat umfasst, deren Stärke zwischen 5 und 200 µm liegt.

13. Pfanne gemäß einem der vorangehenden Ansprüche, **dadurch gekennzeichnet, dass** die erste Schicht aus einer Vielzahl an Titankörnern besteht, die im biologisch verträglichen Polymer individuell versiegelt sind.

14. Pfanne gemäß Anspruch 13, **dadurch gekennzeichnet, dass** die zweite Schicht im Wesentlichen durchgängig ist und im Wesentlichen mit allen Titankörnern der ersten Schicht in Kontakt ist.

15. Verfahren zur Herstellung einer Pfanne nach irgendeinem der Ansprüche 1 bis 14, wobei das Herstellungsverfahren die folgenden Schritte umfasst:
a) eine Pfanne aus einem biologisch verträglichen Material wie beispielsweise UHMPWE oder PEAK steht zur Verfügung;
b) die Pfanne wird mit einer ersten Titanschicht überzogen;
c) die erste Titanschicht wird mit einer zweiten Schicht überzogen, wobei es sich bei der zweiten Schicht um eine Titan- oder Kalziumphosphatschicht handelt;
wobei das Verfahren **dadurch gekennzeichnet ist, dass** die erste Titanschicht durch Heißpressen auf die Pfanne aufgebracht wird, wobei die Rauheit Rt der ersten Titanschicht zwischen 40 und 500 µm liegt.

16. Verfahren zur Herstellung einer Pfanne gemäß Anspruch 15, **dadurch gekennzeichnet, dass** es einen Schritt d) umfasst, bei dem die zweite Schicht, sofern es sich bei dieser zweiten Schicht um eine Titanschicht handelt, mit einer dritten Schicht überzogen wird, wobei es sich bei der dritten Schicht um eine Kalziumphosphatschicht handelt.

17. Verfahren zur Herstellung einer Pfanne gemäß Anspruch 16, **dadurch gekennzeichnet, dass** die dritte Schicht mittels Plasmaspritzen, Tauchlackierung oder Eintauchen in ein Kalziumphosphatbad auf die zweite Schicht aufgetragen wurde.

18. Verfahren zur Herstellung einer Pfanne gemäß einem der Ansprüche 15 bis 17, **dadurch gekennzeichnet, dass** die zweite Schicht eine Kalziumphosphatschicht ist und mittels Plasmaspritzen, Tauchlackierung oder Eintauchen in ein Kalziumphosphatbad auf die erste Schicht aufgetragen wurde.

19. Verfahren zur Herstellung einer Pfanne gemäß einem der Ansprüche 15 bis 17, **dadurch gekennzeichnet, dass** die zweite Schicht eine Titanschicht ist und mittels Plasmaspritzen auf die erste Schicht aufgetragen wurde.

20. Verfahren zur Herstellung einer Pfanne nach einem der Ansprüche 15 bis 19, **dadurch gekennzeichnet, dass** der Schritt b), bei dem die Pfanne mit der ersten Titanschicht überzogen wird, durch Heißpressen einer Vielzahl an Titankörnern auf das biologisch verträgliche Polymer ausgeführt wird, sodass die Körner einzeln im biologisch verträglichen Polymer versiegelt werden.

## Claims

1. An acetabulum for a hip prosthesis made from a biocompatible polymer, said acetabulum being covered with a coating that comprises a first layer of titanium applied on said biocompatible polymer and that further comprises, on the first layer, a second layer of titanium or of a calcium phosphate, **characterized in that**:
- the roughness Rt of the first layer of titanium is comprised between 40 and 500 µm,
- said first layer of titanium has been applied by hot compression on said biocompatible polymer.

2. The acetabulum according to claim 1, **characterized in that** said roughness Rt of the first layer of titanium is comprised between 100 and 500 µm.

3. The acetabulum according to claim 1 or 2, **characterized in that** said coating comprises on said second layer, when the second layer is a layer of titanium, a third layer of a calcium phosphate.

4. The acetabulum according to one of claims 1 to 3, **characterized in that** the biocompatible polymer is chosen in the group consisted by the ultra-high-molecular-weight polyethylene (UHMWPE), non-cross-linked, cross-linked and/or charged with antioxidants preferably chosen among the carotenoids, the vitamins A, B, C, E (tocopherols) and K, the polyphenols, the BHT, the propyl, octyl and dodecyl gallates, the lipoic acid, the dihydroxylipoic acid, the amines, the hydroquinone, the coenzyme Q10 and the glutathione, the polyaryletherketone (PEAK) such as the polyetheretherketones (PEEK), charged or not with carbon.

5. The acetabulum according to any one of the preceding claims, **characterized in that** the thickness of the first layer of titanium is comprised between 50 and 800 µm.

6. The acetabulum according to claim 5, **characterized in that** the thickness of the first layer of titanium is comprised between 150 and 500 µm.

7. The acetabulum according to any one of the preceding claims, **characterized in that** the rate of covering of the first layer of titanium on the biocompatible polymer is at least 60 %.

8. The acetabulum according to any one of the preceding claims, **characterized in that** the roughness of the second layer is comprised between 30 and 400 µm.

9. The acetabulum according to claim 8, **characterized in that** the roughness of the second layer is comprised between 100 and 400 µm.

10. The acetabulum according to any one of the preceding claims, **characterized in that** the thickness of the second layer of the acetabulum coating is comprised between 5 and 500 µm.

11. The acetabulum according to claim 10, **characterized in that** the thickness of the second layer of the acetabulum coating is comprised between 10 and 400 µm.

12. The acetabulum according to any one of claims 1 to 11, **characterized in that** the coating of said acetabulum comprises a second layer of titanium and a third layer of a calcium phosphate whose thickness is comprised between 5 and 200 µm.

13. The acetabulum according to one of the preceding claims, **characterized in that** said first layer is consisted of a multitude of grains of titanium that are individually sealed in the biocompatible polymer.

14. The acetabulum according to claim 13, **characterized in that** said second layer is substantially continuous and is in contact with substantially all the grains of titanium of the first layer.

15. A method for manufacturing an acetabulum according to any one of claims 1 to 14, said manufacturing method comprising the following steps:
a) providing an acetabulum made from a biocompatible material, for example UHMPWE or PEAK;
b) coating said acetabulum with a first layer of titanium;
c) coating said first layer of titanium with a second layer, said second layer being a layer of titanium or a layer of a calcium phosphate;
said method being **characterized in that** said acetabulum is coated with said first layer of titanium by hot compression, the roughness Rt of the first layer of titanium being comprised between 40 and 500 µm.

16. The method for manufacturing an acetabulum according to claim 15, **characterized in that** it comprises a step d) in which, when the second layer is a layer of titanium, said second layer is coated with a third layer, said third layer being a layer of a calcium phosphate.

17. The method for manufacturing an acetabulum according to claim 16, **characterized in that** the third layer has been applied on the second layer by plasma spraying, electroplating, or soaking into a bath of calcium phosphate.

18. The method for manufacturing an acetabulum according one of claims 15 to 17, **characterized in that** the second layer is a layer of a calcium phosphate and has been applied on the first layer by plasma spraying, electroplating, or soaking into a bath of calcium phosphate.

19. The method for manufacturing an acetabulum according one of claims 15 to 17, **characterized in that** the second layer is a layer of titanium and has been applied by plasma spraying on the first layer.

20. The method for manufacturing an acetabulum according one of claims 15 to 19, **characterized in that** the step b) during which said acetabulum is coated with said first layer of titanium is performed by hot compression of a multitude of grains of titanium on said biocompatible polymer, so that said grains are individually sealed in the biocompatible polymer.
